Europäisches Patentamt

European Patent Office

Office européen des brevets

Veröffentlichungsnummer: **0 285 833**
**A2**

# EUROPÄISCHE PATENTANMELDUNG

Anmeldenummer: 88103667.7

Int. Cl.⁴ **G01N 27/18**

Anmeldetag: **09.03.88**

Priorität: **04.04.87 DE 3711511**

Veröffentlichungstag der Anmeldung:
**12.10.88 Patentblatt 88/41**

Benannte Vertragsstaaten:
**AT CH DE FR GB IT LI NL SE**

Anmelder: **Hartmann & Braun**
**Aktiengesellschaft**
**Gräfstrasse 97**
**D-6000 Frankfurt am Main 90(DE)**

Erfinder: **Göldner, Heinz-Dieter, Dr.**
**Ostring 43**
**D-6231 Schwalbach/Ts.(DE)**
Erfinder: **Horn, Bertold, Dr.**
**Lilienweg 15**
**D-6382 Friedrichsdorf(DE)**
Erfinder: **Liedtke, Thomas**
**Hügelstrasse 7**
**D-6370 Oberursel(DE)**
Erfinder: **Marx, Wolf-Rüdiger, Dr.**
**Wiesenblick 17**
**D-6368 Bad Vilbel Gronau(DE)**
Erfinder: **Schaefer, Werner, Dr.**
**Albert Schweitzerstrasse 12**
**D-6242 Kronberg(DE)**

**Verfahren zur Bestimmung der Gaskonzentrationen in einem Gasgemisch und Sensor zur Messung der Wärmeleitfähigkeit.**

Die Erfindung beschreibt ein Verfahren zur Bestimmung der Gaskonzentrationen in einem Gasgemisch durch Messung der Wärmeleitfähigkeit des Gasgemisches. Die Wärmeleitfähigkeit des Gasgemisches mit N Gaskomponenten wird bei N-1 Gastemperaturen gemessen. Aus den ermittelten Wärmeleitfähigkeits-Meßwerten werden die einzelnen Gaskonzentrationen berechnet.

Ein geeigneter Sensor zur Messung der Wärmeleitfähigkeit bei verschiedenen Gastemperaturen besteht aus einer Si-Trägerplatte (1) mit aufgeätzten Dünnfilmwiderständen (5), auf der eine Deckplatte (2) ruht. Beide Platten weisen in Höhe der Dünnfilmwiderstände eingeätzte Gruben (7a, 7b) auf, die die Meßkammer bilden. Über einen Diffusionskanal (8) und Öffnungen (11a, 11b) hat das zu bestimmende Gasgemisch Zutritt zur Meßkammer.

Fig. 1

## Verfahren zur Bestimmung der Gaskonzentrationen in einem Gasgemisch und Sensor zur Messung der Wärmeleitfähigkeit

Die Erfindung betrifft ein Verfahren zur Bestimmung der Gaskonzentrationen in einem Gasgemisch nach dem Oberbegriff des Anspruches 1 sowie einen Sensor zur Durchführung des Verfahrens zur Messung der Wärmeleitfähigkeit eines Gasgemisches nach Anspruch 4.

### Stand der Technik

Es ist allgemein bekannt, die Unterschiede in der Wärmeleitfähigkeit verschiedener Gase zu nutzen, um die Konzentrationen einzelner Gase in Gasgemischen zu bestimmen. Allen Meßverfahren gemeinsam ist, daß die Wärmeleitfähigkeit nur bei einer festen Gastemperatur gemessen wird, bzw. bei einer festen mittleren Gastemperatur, wenn die Meßtemperatur aus Gründen der Signalverarbeitung moduliert wird. Aus prinzipiellen Gründen können dabei nur binäre Gasgemische vollständig bestimmt werden. Durch das Auftreten einer weiteren Gaskomponente bekannter oder unbekannter Gasart, wird das Meßsignal verfälscht.

Die zur Messung der Wärmeleitfähigkeit eingesetzten Analysatoren sind im allgemeinen nach folgendem Prinzip aufgebaut: Ein als Wärmequelle dienendes Widerstandsheizelement wird mittels Stromdurchfluß auf eine gegenüber seiner Umgebung erhöhte Temperatur gebracht. Über eine, durch die Geometrie festgelegte Wärmeleitstrecke wird vom Gas Wärme von der Wärmequelle zu einer auf konstanter Temperatur gehaltenen Wärmesenke geleitet. Durch den Wärmetransport von der Wärmequelle zur Senke wird der Wärmequelle Energie entzogen, die ein Maß für die Wärmeleitfähigkeit des Gases ist und die sich mit geeigneten Verfahren messen Läßt.

Die Wärmeleitung eines Gases ist temperaturabhängig. Um Einflüsse des Temperaturkoeffizienten der Wärmeleitung auszuschalten, werden die Meßzellen thermostatisiert, d.h. durch eine elektronische Regelung auf konstanter Temperatur gehalten. Außer von der Temperatur der Meßzelle wird die mittlere Gastemperatur in der Wärmeleitstrecke von der Temperatur der Wärmequelle bestimmt. Deshalb wird auch diese konstant gehalten oder reproduzierbar eingestellt.

Eine Schwierigkeit bei herkömmlichen Wärmeleitfähigkeitssensoren ist, daß einerseits die Wärmeleitstrecke ständig vom Gas durchströmt werden muß um einen schnellen Gasaustausch zu erreichen und daß andererseits die Strömung des Gases den Wärmetransport und damit den Meßwert beeinflußt. In der DE-OS 35 02 440 wird deshalb ein Verfahren zu Strömungskompensation beschrieben. Auch ist aus der DE-PS 29 52 137 eine Meßzelle bekannt, in der das Gas nicht durch Strömung sondern durch Diffusion ausgetauscht wird. Das Meßkammervolumen und die Gaswege sind jedoch relativ groß, so daß die Gasaustauschzeit in der Meßkammer für bestimmte Meßaufgaben noch zu groß ist.

### Aufgabe der Erfindung

Der Erfindung liegt die Aufgabe zugrunde, ein Verfahren zur Bestimmung das Gaskonzentrationen in einem Gasgemisch von mehr als zwei Gasen anzugeben und einen Sensor zu entwickeln, der eine Gasaustauschzeit von kleiner . Sekunde ausweist und für die Anwendung des Verfahrens geeignet ist.

Die Aufgabe wird erfindungsgemäß mit den in den Ansprüchen 1 und 4 angegebenen Mitteln gelöst.

### Vorteile der Erfindung

Durch die Messung der Wärmeleitfähigkeit eines Gasgemisches bei mehr als einer Gastemperatur ist es möglich, die Konzentrationen einzelner Gase in einem mehr als zwei komponentigen Gasgemisch zu bestimmen, oder das Auftreten einer weiteren, unbekannten Komponente, und damit einen Störfall, zu erkennen.

Insgesamt wird der Einsatzbereich eines Wärmeleitfähigkeitssensors durch das erfindungsgemäße Verfahren wesentlich erweitert.

Besonders vorteilhaft ist das Verfahren anzuwenden, wenn die Messung der Wärmeleitfähigkeit bei verschiedenen Gastemperaturen nicht durch den Einsatz mehrerer Sensoren, die auf verschiedenen Temperaturen gehalten werden, durchgeführt wird, sondern nur ein Sensor benutzt wird. Es wird dadurch zum einen der Aufwand für eine komplette Maßeinheit wesentlich verringert, vor allem aber werden mögliche Fehlerquellen durch unterschiedliche Drift der verschiedenen Sensoren ausgeschlossen.

Voraussetzung für eine Messung mit nur einem Sensor ist, daß die thermische Zeitkonstante des Sensors genügend klein ist und dadurch die Gastemperaturen schnell eingestellt werden können. Durch die äußerst geringe Masse der Heiz-und Meßelemente und der sie tragenden Membran (einige $\mu$g) hat der erfindungsgemäße Sensor eine thermische Zeitkonstante, die im Bereich einer ms liegt. Er erfüllt somit die Voraussetzungen für die Anwendung der Verfahren in besonders vorteilhafter Weise.

Ein weiterer Vorteil des Sensors ist, daß durch die Verwendung einer sehr dünnen Trägerschicht für die Meß-bzw. Heizelemente die Wärmeleitung über die Gase viel größer ist, als die Wärmeableitung über das Material des Heizelementes oder dessen Träger, und somit eine große Empfindlichkeit des Sensors gegenüber einer Änderung der Wärmeleitfähigkeit des Gases erreicht wird. Durch die geringe Wärmeableitung über die dünne Membran kann auch die Wärmeableitung über das Gas durch Wahl einer niedrigen Differenztemperatur zwischen Wärmequelle und Wärmesenke und einer geeigneten Geometrie reduziert werden, wodurch die elektrische Verlustleistung des Sensors für viele Gasgemische unter 10 mW gebracht werden kann. Der Sensor ist damit besonders für die Ansteuerung mit mikroelektronischen Bauelementen geeignet.

Durch die Miniaturisierung des Sensors wird ein Gasvolumen von weniger als 1 $\mu$l erreicht, in welches das Gas wegen der Anordnung der Öffnung zur Meßkammer nur durch Diffusion, nicht aber durch Strömung gelangen kann. Bei geeignet gewähltem Verhältnis von Größe der Diffusionsöffnung und des Kammervolumens werden Gasaustauschzeiten von weniger als 100 ms erreicht, ohne daß eine Gasströmung außerhalb des Sensors den Meßwert beeinflußt.

Der integrierte Temperaturmeßwiderstand ermöglicht eine elektronische Korrektur eventuell auftretender Temperaturschwankungen am Sensor.

Die Passivierung des Sensors mit z.B. $SiO_2$ oder $Si_3N_4$ ermöglicht dessen Einsatz auch in Gasgemischen mit aggressiven Komponenten.

Durch die Verwendung des Siliziums als Ausgangsmaterial und dessen Verarbeitung durch die in der Halbleiterbauelementefertigung bewährten Verfahren wie Sputter-und Aufdampftechnik, Fotolithigraphie, Ätztechnik und Passivierungstechnik ist eine sehr kostengünstige Herstellung von vielen Sensoren aus einem einzigen Silizium-Wafer möglich.

## Beschreibung

Bei den heute bekannten Meßverfahren wird die Messung der Wärmeleitfähigkeit genutzt, um das Verhältnis der Komponenten eines binären Gasgemisches zu bestimmen. Mehr als 2-komponentige Gemische können aus prinzipiellen Gründen durch einen einzigen Meßwert nicht vollständig bestimmt werden. Einen Ausweg aus dieser Situation ergibt sich durch den Umstand, daß die Wärmeleitfähigkeit der Gase im allgemeinen eine Funktion der Gastemperatur ist, und daß dieser funktionelle Zusammenhang auch für die verschiedenen Gase mehr oder weniger unterschiedlich ist. Entsprechend dem Verfahren nach Anspruch 1 ist es deshalb unter bestimmten Voraussetzungen möglich, durch Messung der Wärmeleitfähigkeit eines Gasgemisches bei verschiedenen Gastemperaturen die einzelnen Gaskonzentrationen zu bestimmen.

Da die Wärmeleitfähigkeit der Gase im atmosphärischen Druckbereich (um $10^5$ Pa) im allgemeinen druckunabhängig ist, werden durch die Messung der Wärmeleitfähigkeit des Gemisches nicht die absoluten Konzentrationen, d.h. die Partialdrucke, gemessen, sondern nur deren Verhältnisse. Für die Berechnung von N-1 Verhältnissen von N Gaskomponenten sind deshalb auch nur Messungen bei N-1 verschiedenen Gastemperaturen notwendig.

Weder die Wärmeleitfähigkeit eines Gasgemisches noch die Umsetzung der Wärmeleitfähigkeit in ein elektrisches Signal durch einen Sensor ist im allgemeinen eine lineare Funktion der einzelnen Gaskonzentrationen. Zur Bestimmung der Konzentrationen eines N-komponentigen Gemisches ist deshalb die Lösung eines nicht linearen Gleichungssystems mit N-1 Gleichungen notwendig. (Vergleiche "Transport Phenomena" von R.B. Bird, W.E. Stewart, E.N. Lightfoot, 1960, Kap. 8, S. 243 ff., Herausgeber John Wiley & Sons, New York and London) Ob das Gleichungssystem praktisch lösbar ist, hängt von dem Unterschied in der Temperaturabhängigkeit der Wärmeleitfähigkeit der beteiligten Komponenten, der Anzahl der Komponenten und der Meßgenauigkeit ab. Im allgemeinen wird die Bestimmung von sehr vielen 3-komponentigen und einigen 4-komponentigen Gemischen möglich sein.

Außer der Konzentrationsbestimmung eines N-komponentigen Gasgemisches mit bekannten Komponenten kann die Messung der Wärmeleitfähigkeit bei verschiedenen Gastemperaturen genutzt werden, um in einem normalerweise N-komponentigen Gasgemisch das Auftreten einer N + 1 ten unbekannten Komponente nachzuweisen. Dazu wird geprüft, ob die Meßwerte der Wärmeleitfähigkeit bei zwei verschiedenen Gastemperaturen der bekannten Temperaturabhängigkeit der zwei bekannten Komponenten entspricht. Ist dies nicht der Fall, so wird dadurch das Auftreten einer unbekannten Komponente signalisiert. Das Verfahren funktioniert nur dann nicht, wenn die Temperaturabhängigkeit der unbekannten Komponente gleich der einer der bekannten Komponenten oder gleich einer Linearkombination der Temperaturabhängigkeit der beiden bekannten Komponenten ist. Dies ist jedoch bei einer 3. Komponente in einem binären Gemisch praktisch nie der Fall. Die Nachweisgrenze für eine 2. unbekannte Komponente liegt im allgemeinen weit unter 1 Vol.%. Für das Auftreten einer 4. unbekannten Komponente in einem 3 komponentigen Gemisch steigt die Nachweisgrenze jedoch stark an, so daß praktisch das Auftreten nicht sicher nachgewiesen werden kann.

Ein bevorzugtes Ausführungsbeispiel des Sensors wird anhand der Zeichnung näher erläutert.

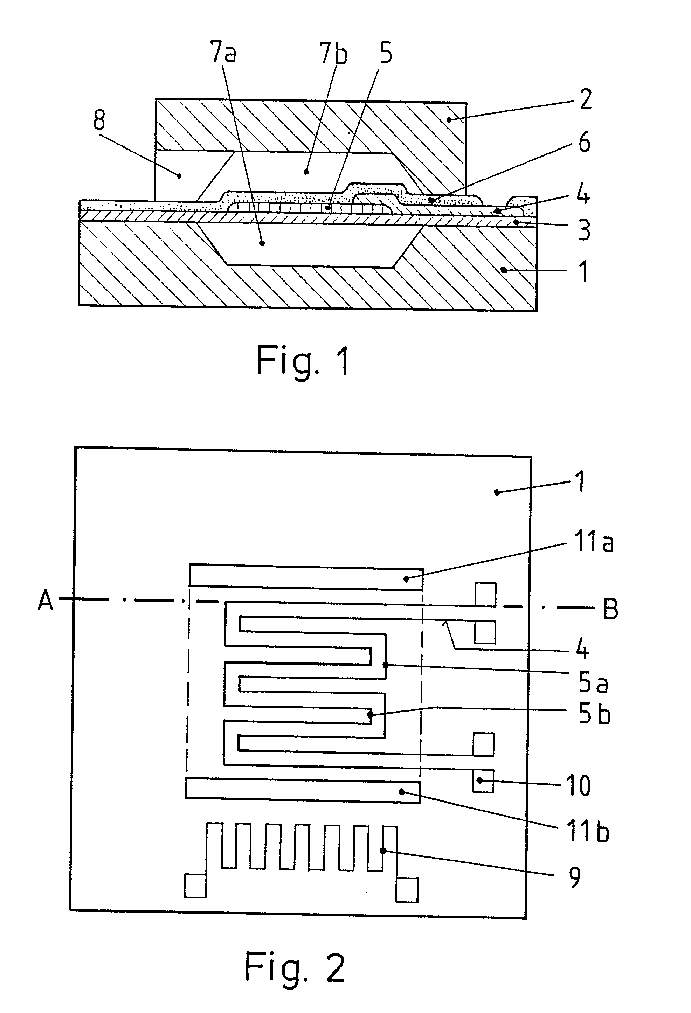

Figur 1 zeigt einen Querschnitt längs der Schnittlinie A-B durch einen Sensor zur Messung der Wärmeleitfähigkeit eines Gases und

Fig. 2 eine Aufsicht auf den Sensor bei abgenommener Deckplatte mit den Meßelementen. Der Sensor besteht aus einer Trägerplatte 1 und einer Deckplatte 2 aus Silizium (Si) mit einer beispielhaften Dicke von je 370 μm, wie sie bei Si-Wafern für Halbleiterbauelemente üblich ist. Die beiden Platten 1 und 2 sind durch Kleben, Löten oder anodisches Bonden miteinander verbunden. Der Sensor hat eine Fläche von etwa 5 × 5 mm² Auf der Trägerplatte 1 ist eine Isolatorschicht 3 aufgebracht, beispielsweise durch das unter der Bezeichnung PECVD bekannte Verfahren. Auf diese Isolatorschicht 3 werden durch Aufdampfen oder Sputtern und nachfolgenden fotolithographischen Methoden mäanderförmige Dünnfilmwiderstände 5, 9, vorzugsweise aus Nickel oder Platin, aufgebracht. Die Dünnfilmwiderstände sind durch Leiterbahnen 4 z.B. aus Gold, die mit den gleichen Verfahren hergestellt werden, mit den Anschlußflächen 10, ebenfalls aus Gold, verbunden, an denen der Sensor kontaktiert werden kann. In Fig. 2 bestehen beispielhaft die Meßelemente des Sensors aus zwei ineinander verschlungenen Dünnfilmwiderständen 5a, 5b, von denen einer als Heizelement, der andere als Temperaturwiderstand genutzt wird. Desweiteren gibt es einen einzelnen Temperaturmeßwiderstand 9.

Nach dem Aufbringen der Meßelemente werden diese durch eine geeignete Beschichtung 6 mit einer Dicke von weniger als 1 μm geschützt.

Unter den Meßelementen in einem Bereich von etwa 1 × 1 mm² ist in die Isolatorschicht bis in eine Tiefe a von etwa 100 μm durch ein anisotropes Ätzmittel, wie z.B. Kalilauge, eine Grube 7a geätzt, die die gewünschte Wärmeleitstrecke bildet. Ebenso ist in die Deckplatte 2 bis in eine Tiefe b von etwa 100 μm eine Grube 7b mit den gleichen Abmessungen geätzt, wobei jedoch eine dünne Isolatorschicht und darauf aufgebrachte Meßelemente fehlen. Die Deckplatte 2 besitzt außerdem eine Öffnung 8, die als Diffusionskanal dem Gas den Zutritt zur Meßkammer, die aus den beiden Gruben 7a und 7b gebildet wird, ermöglicht. Die Abmessungen dieses Diffusionskanals sind dabei so zu wählen, daß einerseits durch eine große Öffnung das Gas in der Meßkammer möglichst schnell durch Diffusion ausgetauscht wird, andererseits aber Gasbewegungen, die außerhalb der Meßkammer auftreten, sich nicht in die Meßkammer übertragen können, was eine kleine Öffnung verlangt. In einer beispielhaften Ausführung des Sensors ist die Höhe der Öffnung 8 80 μm, die Breite und die Länge je 1 mm. Damit werden Gasaustauschzeiten von einigen hundert ms erreicht, ohne daß die Messung der Wärmeleitfähigkeit durch Strömung des Gases außerhalb des Sensors gestört wird. Der Gasaustausch in der unteren Grube 7a der Meßkammer erfolgt durch die Durchbrüche 11a und 11b in der Isolatorschicht 3. Zwecks besserer Unterätzung kann die Isolatorschicht 3 auch weitere in Fig. 2 nicht dargestellte Durchbrüche besitzen.

## Ansprüche

1) Verfahren zur Bestimmung der Gaskonzentrationen in einem Gasgemisch durch Messung der Wärmeleitfähigkeit des Gasgemisches, das aus N Gaskomponenten mit N größer 2 besteht, gekennzeichnet dadurch, daß die Wärmeleitfähigkeit des Gasgemisches bei mindestens N-1 Gastemperaturen gemessen wird und daß aus den ermittelten Wärmeleitfähigkeits-Meßwerten mit bekannten mathematischen Methoden zur Lösung nichtlinearer Gleichungssysteme die einzelnen Gaskonzentrationen berechnet werden.

2) Verfahren nach Anspruch 1 zur Erkennung des Auftretens unbekannter Gaskomponenten in einem Gasgemisch aus N bekannten Gaskomponenten, gekennzeichnet dadurch, daß die Wärmeleitfähigkeit des Gasgemisches bei mindestens N Gastemperaturen gemessen wird und daß aus den ermittelten Wärmeleitfähigkeits-Meßwerten das Vorhandensein unbekannter Gaskomponenten mit bekannten mathematischen Methoden zur Lösung nichtlinearer Gleichungssysteme bestimmbar ist.

3) Verfahren nach Anspruch 1 oder Anspruch 2 gekennzeichnet dadurch, daß die Wärmeleitfähigkeit bei verschiedenen Gastemperaturen mit nur einem Wärmeleitfähigkeits-Sensor gemessen wird, bei dem jede Gastemperatur in einer Zeit von weniger als 1 Sekunde zyklisch einstellbar ist.

4) Sensor zur Messung der Wärmeleitfähigkeit eines Gasgemisches, insbesondere zur Durchführung eines Verfahrens nach Anspruch 1, 2 oder Anspruch 3, gekennzeichnet dadurch, daß der Sensor folgende Konstruktionsmerkmale aufweist:
- auf einer einige 100 μm starken Trägerplatte (1) aus Silizium ist eine Isolatorschicht (3) aufgetragen, auf der durch Aufdampfen oder Sputtern mäanderförmige Dünnfilmwiderstände (5a, 5b) aufgebracht sind,

- im Bereich der Dünnfilmwiderstände (5a, 5b) ist die Isolatorschicht (3) unterätzt, so daß eine Grube (7a) in der Trägerplatte (1) entsteht, die den unteren Teil der Meßkammer des Sensors bildet,

- auf der Trägerplatte (1) mit den Dünnfilmwiderständen (5a, 5b) ruht eine Deckplatte (2) aus Silizium, in die in Höhe der Dünnfilmwiderstände (5a, 5b) eine Grube (7b) eingeätzt ist, die den oberen Teil der Meßkammer bildet und

- die Deckplatte (2) besitzt eine Öffnung (8), die als Diffusionskanal dem Gasgemisch den Zutritt zur Meßkammer (7a, 7b) ermöglicht.

5) Sensor nach Anspruch 4, gekennzeichnet dadurch, daß die Isolatorschicht (3) aus Siliziumoxyd ($SiO_2$) oder aus Siliziumnitrid ($Si_3N_4$), oder aus einer Mischung aus beiden Silizium-Verbindungen oder aus einer mehrlagigen Schicht der beiden Silizium-Verbindungen besteht.

6) Sensor nach Anspruch 4, gekennzeichnet dadurch, daß auf der Trägerplatte (1), außerhalb der Meßkammer (7a, 7b), ein Temperaturmeßwiderstand (9) aufgebracht ist.

7) Sensor nach Anspruch 4, gekennzeichnet dadurch, daß die Dünnfilmwiderstände (5a, 5b) durch eine Schicht mit einer Dicke von ca. 1 $\mu$m, vorzugsweise aus Siliziumoxyd ($SiO_2$) oder Siliziumnitrid ($Si_3N_4$), gegen chemische Beeinflussung du@ch das Gasgemisch geschützt sind.

8) Sensor nach Anspruch 4, gekennzeichnet dadurch, daß die Isolatorschicht (3) Öffnungen (11a, 11b) aufweist, durch die der Gasaustausch im unteren Teil (7a) der Meßkammer erfolgt.

9) Sensor nach Anspruch 4, gekennzeichnet dadurch, daß die Grube (7b) in der Deckplatte (2) eine Tiefe von 10 bis 100 $\mu$m aufweist.

Fig. 1

Fig. 2